# FASCICULE DE BREVET EUROPEEN

(11) **EP 0 806 949 B1**
(45) Date de publication et mention de la délivrance du brevet: **06.08.2003**
(21) Numéro de dépôt: 96903042.8
(22) Date de dépôt: 02.02.1996
(51) Int. Cl.: A61K 31/24, A61K 9/00

(54) **NOUVELLE COMPOSITION PHARMACEUTIQUE CONTENANT LA TRIMEBUTINE ET SON PROCEDE DE PREPARATION**
TRIMEBUTINHALTIGES ARZNEIMITTEL UND VERFAHREN
NEW PHARMACEUTICAL COMPOSITION CONTAINING TRIMEBUTINE AND PREPARATION PROCESS

(30) Priorité: 03.02.1995 FR 9501289
(43) Date de publication de la demande: 19.11.1997
(73) Titulaire: Pfizer Holding France, 75014 Paris (FR)
(72) Inventeur: DOAT, Bernard, F-49000 Angers (FR)
(74) Mandataire: Hirsch, Denise Marie
(86) Numéro de dépôt international: FR9600179
(87) Numéro de publication internationale: WO96023493

(56) Documents cités:
- EP-A- 0 076 515
- FR-A- 2 640 876
- DATABASE WPI Week 9204 Derwent Publications Ltd., London, GB; AN 92-028855 XP002003118 & JP,A,03 275 622 (TEISAN SEIYAKU KK) 6 Décembre 1991

## Description

### Domaine de l'invention

La présente invention a pour objet une composition pharmaceutique dont le principe actif est la trimébutine sous forme de poudres ou de granulés selon les revendications 1 à 9 ainsi que leur procédé de préparation.

### Arrière-plan technologique de l'invention

La trimébutine, ou 3,4,5-triméthoxybenzoate de 2-(diméthylamino)-2-phényl-*n*-butyle, est le premier médicament agissant sur les récepteurs enképhalinergiques périphériques, notamment digestifs. C'est un régulateur de la motricité digestive. Telle qu'elle, ou salifiée par l'acide maléique, la trimébutine est proposée sous forme de comprimé, de solution injectable, de suppositoire et de poudre pour préparation d'une suspension buvable; en outre une forme à libération prolongée a fait l'objet du brevet français n' 2 640 876.

Internationalement commercialisées, les formes orales de trimébutine sont indiquées dans le traitement des douleurs liées aux troubles fonctionnels du tube digestif et des voies biliaires, et dans le traitement des douleurs et de l'inconfort liés aux troubles fonctionnels intestinaux.

Parmi les formes orales proposées, la suspension buvable est celle plus particulièrement adaptée à l'usage pédiatrique. Jusqu'alors ce produit est présenté dans un flacon de verre sous la forme d'une poudre colorée et aromatisée à laquelle on doit ajouter un volume précis d'eau, pour, après homogénéisation énergique, obtenir la suspension prête à l'emploi. Le conditionnement unitaire courant est un flacon de 152,5 g de poudre contenant 1,2 g de trimébutine dans lequel on ajoute une quantité d'eau suffisante pour obtenir après mélange 250 ml de suspension buvable. L'unité de mesure indiquée pour l'administration aux patients est la cuillère, à soupe pour les adultes, à café pour les enfants, les posologies pour les plus jeunes étant déterminées en fractions de cuillères.

A l'usage, cette suspension buvable s'avère peu pratique pour une utilisation impromptue et plus généralement lors d'un traitement ambulatoire, et ce aussi bien à cause de l'indispensable préparation préliminaire que par son flaconnement encombrant et fragile. Egalement, de par les unités de mesures préconisées, les posologies sont peu précises (variabilité de la contenance des cuillères), et leur administration pose problème pour le traitement de jeunes enfants.

Dans cette préparation aqueuse, la trimébutine, dont la structure comporte une fonction ester hydrolysable, est pratiquement insoluble et est à l'état de dispersion dans le soluté aqueux.

Sans pour autant l'éviter totalement, la forme suspension, dans des conditions de conservation normales, minimise au mieux l'hydrolyse du principe actif. Une période limite d'utilisation à compter de la préparation de la suspension est cependant indiquée.

Cet état de fait peut entraîner le gaspillage de produit lorsque le traitement est interrompu précocement ou lors du traitement de jeunes enfants pour lesquels la posologie indiquée est faible; rapidement, dans ces deux cas, l'hydrolyse de la trimébutine entraîne l'inefficacité du restant de la suspension qui devient alors inutilisable sinon dangereuse puisqu'elle s'avère incapable de s'opposer à l'évolution d'une pathologie.

La non observance des conseils d'utilisation de la suspension, notamment une mauvaise homogénéisation avant emploi, peut avoir de graves conséquences. Ainsi, au début du traitement, le prélèvement d'une suspension non homogène d'un flacon plein, dans lequel la trimébutine sera décantée, n'entraînera pas l'effet thérapeutique désiré; par contre, en fin de traitement, les prélèvements seront surdosés en principe actif et seraient susceptibles de provoquer des effets secondaires.

Une solution palliative à ces problèmes consiste à répartir la poudre en doses unitaires, par exemple dans des sachets afin de faciliter leur utilisation dans les traitements ambulatoires. Sur la base posologique indiquée, qui est conventionnellement de 15 ml pour une cuillère à soupe, le contenu d'un flacon de 250 ml représente environ 16 doses unitaires d'un poids approximatif de 9,5 g chaque, contenant, selon la formulation en cours, près de 9,2 g de saccharose.

Outre l'apport calorique important qui est d'environ 37 Kcal par dose, une telle quantité de saccharose peut interdire ce traitement aux patients diabétiques. Egalement la faible densité apparente de la poudre pose problèmes au niveau des volumes de conditionnement pour les sachets. En outre, telle que présentée, la poudre ne peut être absorbée sans eau par le patient; il est nécessaire de la diluer dans le minimum d'eau pour reconstituer une suspension buvable. Cette opération s'avère aléatoire compte tenu du saccharose, souvent présent sous forme de cristaux de taille importante enrobés de trimébutine, et qui, en ne se dissolvant que très difficilement, ont pour conséquence de sédimenter et de diminuer la quantité de médicament en suspension et donc absorbée par le patient.

### Description de l'invention

Remédiant à ces problèmes, la présente invention a pour but de fournir une composition, stable et d'apport calorique modéré, contenant la trimébutine et destinée à la préparation de poudres et de granulés pouvant être répartis en doses unitaires afin de faciliter les traitements ambulatoires.

Selon cet aspect, la composition comprend une quantité thérapeutiquement efficace de trimébutine associée avec :
i) un mélange diluant et édulcorant d'apport calorique modéré,
ii) des excipients appropriés à la réalisation d'une suspension aqueuse stable,
iii) des excipients appropriés à la préparation de la composition et à sa manipulation,
iv) des adjuvants d'aromatisation et de coloration adaptés à une présentation agréable de la composition et de la suspension buvable.

Un second aspect de la revendication 9 vise le procédé de préparation de la composition qui consiste essentiellement à mélanger intimement la trimébutine avec les divers constituants, puis à pratiquer une granulation humide du mélange, à sécher les granulés puis à les tamiser avant de les homogénéiser finalement avec l'excipient approprié pour la manipulation aisée destinée au conditionnement du produit ainsi préparé.

Pour la facilité d'utilisation, notamment en traitements ambulatoires, la composition est répartie en conditionnements unitaires, de préférence en sachets, les dits sachets pouvant être sous-compartimentés afin de correspondre aux fractions posologiques inférieures indiquées pour le traitement des enfants.

Dans le cadre du présent mémoire on entend préciser que la composition, selon le procédé mis en oeuvre et notamment la sélection granulométrique effectuée lors de l'opération de tamisage, peut se présenter sous l'aspect de granulés ou de poudres plus ou moins fines qui, en tout état de cause sont destinés à être mis en suspension dans l'eau ou encore dans un liquide ou semi liquide alimentaire, ou bien encore et en dernier recours à être ingérés tels quels.

Qualitativement, la mise en oeuvre du procédé de l'invention est réalisée à partir de trimébutine qui se présente sous la forme d'une poudre cristalline blanche dont la granulométrie est comprise entre 1 micron et 200 microns et plus favorablement de 1 à 50 microns.

Le mélange diluant sert également à édulcorer la composition. Il est constitué de carbohydrates naturels ou de synthèse et d'un agent de synthèse à haut pouvoir sucrant.

Ainsi on utilise des mélanges comprenant un monosaccharide ou un disaccharide comme le saccharose qui est préféré dont on renforce l'effet sucrant par l'addition d'un édulcorant de synthèse comme la saccharine ou l'un de ses sels ou plus favorablement comme l'aspartam.

Les excipients appropriés à la réalisation de la suspension aqueuse stable ont pour but essentiel d'assurer la dispersion homogène de la trimébutine insoluble et son maintien en suspension pendant une durée raisonnable compte tenu du mode d'utilisation du produit. A cet effet les adjuvants de viscosité hydrosolubles ont une action favorable; des épaississants tels que des dérivés cellulosiques comme la carboxyméthylcellulose sodique, l'hydroxypropylméthylcellulose ou bien encore les gommes peuvent être utilisées. La gomme xanthane est préférée, elle constitue le meilleur compromis entre une rapidité de mise en suspension qui est indispensable mais qui est ralentie par un effet visqueux excessif, et le maintien en suspension des particules qui est souhaité et qui, inversement est favorisé par l'augmentation de viscosité.

En ce qui concerne les excipients appropriés à la préparation de la composition et à sa manipulation, on entend d'une part des agents mouillants nécessaires de par le caractère hydrophobe du principe actif et aussi des produits qui favorisent l'opération de granulation et, d'autre part, des produits antistatiques qui favorisent l'écoulement des poudres ou granulés et permettent ainsi le remplissage régulier des unités de conditionnement. Pour le mouillage de la trimébutine on utilise un agent tensio actif pharmaceutiquement compatible que l'on peut choisir par exemple parmi ceux présentés à la liste de USP Ed.XXI p. 1492; parmi ceux- ci le polysorbate 80 est préféré. Pour l'opération de granulation humide, l'addition d'agents liants favorise l'agglomération des particules entre elles et contribue à l'homogénéité de la trimébutine dans la préparation. A cette fin on peut utiliser les composés cellulosiques ou les gommes mentionnés précédemment. Parmi ces dernières la gomme arabique est préférée. La silice colloïdale anhydre est retenue comme agent antistatique qui aide au bon écoulement du produit terminé.

Pour ce qui est des adjuvants de coloration et d'aromatisation, un libre choix peut être fait dans les produits autorisés par la législation. Toutefois l'arôme naturel d'orange associé au colorant Jaune orangé S sont préférés; ils permettent une présentation satisfaisante à une majorité de patients.

Dans son aspect le plus large une composition conforme à la présente invention peut contenir, exprimé en parties pour 10 parties de trimébutine :
- de 40 à 60 parties de mélange édulcorant pouvant être composé de 39,5 à 59,0 parties de saccharose et de 0,5 à 5 parties d'aspartam,
- de 1 à 5 parties de gomme xanthane,
- de 1,6 à 6,5 parties pour le total des excipients utiles à la préparation et la manipulation de la composition, et comprenant de 0,25 à 2,0 parties de polysorbate 80, de 1,0 à 4,0 parties de gomme arabique et de 0,1 à 1,0 partie de silice colloïdale anhydre,
- et de 5 à 40 parties d'arôme naturel d'orange avec de 0,005 à 0,1 partie de colorant Jaune orangé S.

Plus précisément l'invention se rapporte aux compositions qui, favorablement, pour 10 parties de trimébutine, ont la composition suivante :
- de 47,5 à 52,5 parties de mélange édulcorant pouvant être composé de 46,5 à 51,5 parties de saccharose et de 1 à 3 parties d'aspartam,
- de 2,5 à 4 parties de gomme xanthane,
- de 3,5 à 4,5 parties pour le total des excipients utiles à la préparation et la manipulation de la composition, et comprenant de 0,75 à 1,25 parties de polysorbate 80, de 2 à 3 parties de gomme arabique et de 0,4 à 0,7 partie de silice colloïdale anhydre,
- et de 15 à 25 parties d'arôme naturel d'orange avec de 0,01 à 0,03 partie de colorant Jaune orangé S.

Au sens le plus précis de l'invention, les constituants de la composition particulièrement préférée exprimés pour 100g de mélange et pour le contenu d'un sachet dose de 4,00g sont rapportés au tableau suivant :

| Constituants | Composition / 100g | Composition / sachet 4,0g |
|---|---|---|
| - trimébutine | 1,860 g | 0,0744 g |
| - aspartam | 0,375 g | 0,0150 g |
| - arôme naturel orange | 4,000 g | 0,1600 g |
| - gomme xanthane | 0,600 g | 0,0240 g |
| - Jaune orangé S | 0,004 g | 0,00016 g |
| - polysorbate 80 | 0,200 g | 0,0080 g |
| - gomme arabique | 0,450 g | 0,0180 g |
| - silice colloïdale anhydre | 0,100 g | 0,0040 g |
| - saccharose | 92,410 g | 3,6964 g |
| - apport calorique | env. 375 Kcal. | env. 15 Kcal. |

Sans pour autant limiter la portée de l'invention, la préparation de la composition particulièrement préférée est rapportée à la description qui suit;

### Exemple :

**a)** Dans un récipient contenant 270 g d'eau potable on dissout sous agitation mécanique:
   - 0,72 g de Jaune orangé S,
   - 36,00 g de polysorbate 80,
   - 81,00 g de gomme arabique.
**b)** Dans un mélangeur granulateur de type Lodige et d'une contenance de 501 on mélange durant 3 minutes :
   - 337,5 g de trimébutine,
   - 67,5 g d'aspartam,
   - 720,0 g d'arôme orange,
   - 108,0 g de gomme xanthane,
   - 16630 g de saccharose.
**c)** Le mélange est humidifié avec la solution liante et colorante préparée en a),
**d)** Le granulé humide est séché en lit d'air fluidisé à une température de 60°C jusqu'à obtention d'une humidité résiduelle inférieure à 1%,
**e)** Le granulé séché est tamisé sur tamis d'ouverture de maille de 1 mm,
**f)** Le granulé tamisé est homogénéisé avec 18,0 g de silice colloïdale anhydre dans un mélangeur à projection et tourbillonnement ou dans un mélangeur type "container",
**g)** La composition granulée est répartie en sachets (papier/aluminium/polyéthylène) à raison de 4,00 g par unité.

Le contenu d'un sachet, tel que préparé, est ajouté à un quart de verre d'eau. Par une légère agitation on obtient rapidement une suspension dans laquelle les particules insolubles ne sédimentent que très lentement.

Par ailleurs les études de stabilité des granulés conditionnés en sachets, réalisées après 18 mois de conservation à 25°C ou après 6 mois à 37°C ne montrent aucune modification quant à l'aspect du produit et sa dispersibilité dans l'eau. Egalement il n'est observé, pour ces produits mis en étude de stabilité aucune dégradation significative de la trimébutine, notamment recherchée par la présence de ses éventuels composés d'hydrolyse et/ou d'isomérisation.

## Revendications

1. Composition pharmaceutique sous forme de poudre ou de granulés pour administration par voie orale contenant un mélange de trimébutine et des excipients, **caractérisée en ce qu'**un ou plusieurs excipients sont des adjuvants de viscosité hydrosolubles choisis parmi le groupe comprenant les dérivés de la cellulose et les gommes.

2. Composition selon la revendication 1, **caractérisée en ce que** le ou les adjuvants de viscosité hydrosolubles sont choisis parmi le groupe comprenant les gommes.

3. Composition selon la revendication 1, **caractérisée en ce que** les dérivés de la cellulose sont choisis parmi le groupe comprenant la carboxyméthylcellulose sodique et l'hydroxypropylméthylcellulose.

4. Composition selon la revendication 2, **caractérisée en ce que** la gomme est la gomme xanthane.

5. Composition selon la revendication 4, **caractérisée en ce qu'**elle contient de 1 à 5 parties de gomme xanthane pour 10 parties de trimébutine.

6. Composition selon l'une des revendications 1 à 5, **caractérisée en ce que** les excipients comprennent :
- un mélange édulcorant et diluant d'apport calorique modéré,
- des excipients appropriés à la réalisation d'une suspension aqueuse stable,
- des excipients appropriés à la préparation de la composition et à sa manipulation,
- des adjuvants d'aromatisation et de coloration.

7. Composition selon l'une des revendications 1 à 6 **caractérisée en ce que** pour 10 parties de trimébutine elle comprend :
- de 40 à 60 parties de mélange édulcorant pouvant être composé de 39,5 à 59,0 parties de saccharose et de 0,5 à 5 parties d'aspartam,
- de 1 à 5 parties de gomme xanthane,
- de 1,6 à 6,5 parties pour le total des excipients utiles à la préparation et la manipulation de la composition, et comprenant de 0,25 à 2,0 parties de polysorbate 80, de 1,0 à 4,0 parties de gomme arabique et de 0,1 à 1,0 partie de silice colloïdale anhydre,
- et de 5 à 40 parties d'arôme naturel d'orange avec de 0,005 à 0,1 partie de colorant Jaune orangé S.

8. Composition selon l'une des revendications 1 à 7 **caractérisée en ce que** sous forme de poudre ou de granulés elle est conditionnée en sachets doses optionnellement compartimentés.

9. Composition selon l'une des revendications 7 ou 8 **caractérisée en ce qu'**un sachet contient 4,0 g de granulés dont la composition est :
- trimébutine 0,0744 g
- aspartam 0,0150 g
- arôme naturel orange 0,1600 g
- gomme xanthane 0,0240 g
- Jaune orangé S 0,00016 g
- polysorbate 80 0,0080 g
- gomme arabique 0,0180 g
- silice colloïdale anhydre 0,0040 g
- saccharose 3,6964 g.

10. Procédé de préparation de la composition selon l'une des revendications 1 à 9, **caractérisé en ce qu'**il consiste :
- à mélanger intimement la trimébutine avec les divers constituants,
- puis à pratiquer une granulation humide du mélange,
- à sécher les granulés puis les tamiser,
- et à homogénéiser finalement les granulés avec l'excipient approprié à la manipulation pour le conditionnement du produit.

## Patentansprüche

1. Pharmazeutische Zusammensetzung in Form von Pulver oder Granulat zur oralen Verabreichung enthaltend ein Gemisch von Trimebutin und Hilfsstoffen, **dadurch gekennzeichnet, dass** ein oder mehrere Hilfsstoffe wasserlösliche Viskositätsadjuvantien sind, die ausgewählt sind aus der Gruppe, die die Cellulosederivate und die Gummis umfasst.

2. Zusammensetzung gemäß Anspruch 1, **dadurch gekennzeichnet, dass** das oder die wasserlöslichen Viskositätsadjuvantien ausgewählt sind aus der Gruppe, die die Gummis umfasst.

3. Zusammensetzung gemäß Anspruch 1, **dadurch gekennzeichnet, dass** die Cellulosederivate ausgewählt sind aus der Gruppe, die Natriumcarboxymethylcellulose und Hydroxypropylmethylcellulose umfasst.

4. Zusammensetzung gemäß Anspruch 2, **dadurch gekennzeichnet, dass** der Gummi Xanthangummi ist.

5. Zusammensetzung gemäß Anspruch 4, **dadurch gekennzeichnet, dass** sie 1 bis 5 Teile Xanthangummi auf 10 Teile Trimebutin enthält.

6. Zusammensetzung gemäß einem der Ansprüche 1 bis 5, **dadurch gekennzeichnet, dass** die Hilfsstoffe umfassen:
- ein Gemisch Süßstoff und Verdünnungsmittel mit gemäßigter Kalorienzufuhr,
- Hilfsstoffe, die zur Herstellung einer stabilen wässrigen Suspension geeignet sind,
- Hilfsstoffe, die zur Herstellung der Zusammensetzung und zu ihrer Handhabung geeignet sind,
- Adjuvantien zum Aromatisieren und Färben.

7. Zusammensetzung gemäß einem der Ansprüche 1 bis 6, **dadurch gekennzeichnet, dass** sie auf 10 Teile Trimebutin umfasst:
- 40 bis 60 Teile Süßstoffgemisch, das aus 39,5 bis 59,0 Teilen Saccharose und 0,5 bis 5 Teilen Aspartam zusammengesetzt sein kann,
- 1 bis 5 Teile Xanthangummi,
- 1,6 bis 6,5 Teile insgesamt an Hilfsstoffen für die Herstellung und Handhabung der Zusammensetzung, die 0,25 bis 2,0 Teile Polysorbat 80, 1,0 bis 4,0 Teile Gummi arabicum und 0,1 bis 1,0 Teile wasserfreies hochdisperses Siliciumdioxid umfassen,
- und 5 bis 40 Teile natürliches Orangenaroma mit 0,005 bis 1 Teilen Farbstoff Gelborange S.

8. Zusammensetzung gemäß einem der Ansprüche 1 bis 7, **dadurch gekennzeichnet, dass** sie in Form von Pulver oder Granulat in wahlweise unterteilte Dosisbeutel abgepackt ist.

9. Zusammensetzung gemäß einem der Ansprüche 7 oder 8, **dadurch gekennzeichnet, dass** ein Beutel 4,0 g Granulat enthält, dessen Zusammensetzung ist:
- Trimebutin 0,0744 g
- Aspartam 0,0150 g
- natürliches Orangenaroma 0,1600 g
- Xanthangummi 0,0240 g
- Gelborange S 0,00016 g
- Polysorbat 80 0,0080 g
- Gummi arabicum 0,0180 g
- wasserfreies hochdisperses Siliciumdioxid 0,0040 g
- Saccharose 3,6964 g.

10. Verfahren zur Herstellung der Zusammensetzung gemäß einem der Ansprüche 1 bis 9, **dadurch gekennzeichnet, dass** es darin besteht:
- Trimebutin innig mit den verschiedenen Bestandteilen zu mischen,
- dann das Gemisch feucht zu granulieren,
- das Granulat zu trocknen, dann zu sieben,
- und schließlich das Granulat mit dem Hilfsstoff, der zur Handhabung für das Verpacken des Produkts geeignet ist, zu homogenisieren.

## Claims

1. Pharmaceutical composition in the form of powder or granules for oral administration, containing a mixture of trimebutine and excipients, **characterized in that** one or more excipients are water-soluble viscosity adjuvants chosen from the group comprising cellulose derivatives and gums.

2. Composition according to Claim 1, **characterized in that** the water-soluble viscosity adjuvant(s) is (are) chosen from the group comprising gums.

3. Composition according to Claim 1, **characterized in that** the cellulose derivatives are chosen from the group comprising sodium carboxymethylcellulose and hydroxypropylmethylcellulose.

4. Composition according to Claim 2, **characterized in that** the gum is xanthan gum.

5. Composition according to Claim 4, **characterized in that** it contains 1 to 5 parts of xanthan gum per 10 parts of trimebutine.

6. Composition according to one of Claims 1 to 5, **characterized in that** the excipients comprise:
- a sweetening and diluent mixture supplying a moderate caloric intake,
- excipients suitable for the production of a stable aqueous suspension,
- excipients suitable for the preparation of the composition and for manipulation thereof,
- flavouring and colouring adjuvants.

7. Composition according to one of Claims 1 to 6, **characterized in that** it comprises, per 10 parts of trimebutine:
- 40 to 60 parts of sweetening mixture which can be composed of 39.5 to 59.0 parts of sucrose and 0.5 to 5 parts of aspartame,
- 1 to 5 parts of xanthan gum,
- 1.6 to 6.5 parts for the collective excipients which are useful for the preparation and manipulation of the composition, and comprising 0.25 to 2.0 parts of polysorbate 80, 1.0 to 4.0 parts of gum arabic and 0.1 to 1.0 part of anhydrous colloidal silica,
- and 5 to 40 parts of natural orange flavour with 0.005 to 0.1 part of orange yellow S dye.

8. Composition according to one of Claims 1 to 7, **characterized in that**, in powder or granule form, it is packaged in dose sachets which are optionally compartmentalized.

9. Composition according to either of Claims 7 and 8, **characterized in that** one sachet contains 4.0 g of granules, the composition of which is:
- trimebutine 0.0744 g
- aspartame 0.0150 g
- natural orange flavour 0.1600 g
- xanthan gum 0.0240 g
- orange yellow S 0.00016 g
- polysorbate 80 0.0080 g
- gum arabic 0.0180 g
- anhydrous colloidal silica 0.0040 g
- sucrose 3.6964 g.

10. Process for preparing the composition according to one of Claims 1 to 9, **characterized in that** it consists in:
- mixing trimebutine thoroughly with the various constituents,
- then performing a wet granulation of the mixture,
- drying the granules and then sieving them,
- finally homogenizing the granules with the excipient suitable for the manipulation for packaging of the product.
